Europäisches Patentamt

**European Patent Office**

Office européen des brevets

(11) Numéro de publication: **0 254 612 B1**

# FASCICULE DE BREVET EUROPEEN

(45) Date de publication de fascicule du brevet:
12.06.91

(51) Int. Cl.5: **A61K 7/035**

(21) Numéro de dépôt: 87401470.7

(22) Date de dépôt: **25.06.87**

(54) **Compositions sous forme de poudre non compactée contenant des microsphères creuses à base de matériau synthétique thermoplastique.**

(30) Priorité: 26.06.86 FR 8609289

(43) Date de publication de la demande:
**27.01.88 Bulletin 88/04**

(45) Mention de la délivrance du brevet:
**12.06.91 Bulletin 91/24**

(84) Etats contractants désignés:
**DE FR**

(56) Documents cités:
**DE-A- 2 624 289**
**DE-B- 2 521 003**
**US-A- 4 089 800**

**CHEMICAL ABSTRACTS, vol. 104, no. 4, janvier 1986, résumé no. 24076w, Columbus, Ohio, US; & JP-A-60 184 004 (POLA CHEMICAL INDUSTRIES, INC.) 02-03-1984 * & PATENT ABSTRACTS OF JAPAN, vol. 10, no. 31 (C-327)[2088], 6 février 1986; & JP-A-60 184 004 (POLA KASEI KOGYO K.K.) 19-09-1985**

(73) Titulaire: **L'OREAL**
**14, Rue Royale**
**F-75008 Paris(FR)**

(72) Inventeur: **Arraudeau, Jean-Pierre**
**48 rue des Moines**
**F-75017 Paris(FR)**
Inventeur: **Boelle, Anne**
**107 rue de Paris**
**F-92190 Meudon(FR)**

(74) Mandataire: **Nony, Michel et al**
**Cabinet Nony 29, rue Cambacérès**
**F-75008 Paris(FR)**

## Description

La présente invention a pour objet l'utilisation, dans la préparation de poudres non compactées pour le maquillage ou les soins du corps ou du visage, d'un matériau synthétique thermoplastique sous la forme de microsphères creuses de faible densité, ainsi que des compositions sous forme de poudre non compactée contenant un tel matériau.

On sait que certaines compositions pour le maquillage ou les soins de la peau du visage ou du corps se présentent sous la forme de poudres non compactées, encore appelées "poudres libres". Ce sont notamment des poudres de maquillage, des poudres désodorisantes, ou encore des poudres pour les soins des bébés.

De telles poudres doivent posséder plusieurs types de propriétés.

Elles doivent pouvoir absorber l'eau et éventuellement les matières huileuses exsudées par la peau. Ce pouvoir absorbant doit cependant être limité pour ne pas causer une impression de desséchement. En outre, elles ne doivent pas s'agglomérer sous l'effet de l'humidité ambiante.

Elles doivent être dépourvues de tout caractère abrasif, et même posséder un toucher onctueux.

Elles doivent aussi pouvoir être appliquées facilement, tout en adhérant de façon suffisante à la peau.

En outre, les poudres de maquillage du visage doivent avoir un bon pouvoir couvrant, c'est-à-dire permettre de dissimuler les défaut mineurs de la peau, mais leur opacité doit être limitée pour éviter l'obtention d'un aspect "farineux" sur la peau.

Enfin, les poudres doivent être compatibles avec la présence d'une certaine quantité d'huile, qui améliore le confort d'application, et qui peut servir de véhicule pour des ingrédients actifs divers (filtres UV, parfums, vitamines, agents adoucissants, etc...).

Il n'existe aucun matériau pulvérulent connu possédant cet ensemble de propriétés qui, comme on peut le voir aisément d'après les indications qui précèdent, sont quelque peu contradictoires, et les difficultés d'élaboration de telles poudres sont résolues par la réalisation de mélanges de divers ingrédients qui permettent de trouver un équilibre entre toutes les propriétés souhaitées.

Dans la demande de brevet JP-A-60/184 004, on a décrit des poudres cosmétiques contenant des microsphères creuses en matériau thermoplastique, ayant des masses spécifiques de 0,6 à 0,8 g/cm³. De telles microsphères creuses ont un pouvoir limité d'absorption des huiles.

On a maintenant découvert qu'il est possible de réaliser des poudres non compactées fines et légères, peu sensibles à l'humidité ambiante, ayant un toucher onctueux, pouvant comporter des proportions relativement élevées d'huiles sans avoir tendance à s'agglomérer, faciles à étaler et donnant à l'utilisateur une sensation de confort, en incorporant, dans les poudres non compactées pour le maquillage ou les soins du visage ou du corps, un matériau synthétique thermoplastique présenté sous la forme de microsphères creuses de très faible densité.

La préparation de telles microsphères creuses est connue. Elle peut être effectuée par exemple selon les procédés décrits dans le brevet US n° 3.615.972 et la demande de brevet européen n° 0056219.

La partie creuse des microsphères contient un gaz tel qu'un fréon ou un hydrocarbure (butane, pentane) ou tout autre gaz utilisé classiquement.

Une caractéristique importante des microsphères creuses qui sont utilisées selon l'invention est qu'elles ont été obtenues par un procédé comprenant une étape d'expansion à chaud, par exemple dans un atomiseur, comme décrit dans la demande de brevet européen n° 0056219. Cela permet d'obtenir des microsphères creuses de très faible densité, ayant une masse spécifique inférieure à 0,1g/cm³, et même, de préférence, inférieure à 0,05g/cm³, de sorte que des proportions pondérales de l'ordre de 3% correspondent à des proportions en volume de l'ordre de 50%. On a constaté que, de façon surprenante, ces microsphères, lorsqu'elles sont mélangées avec les particules plus denses des autres constituants, permettent d'obtenir des poudres qui restent homogènes : on n'observe pas de ségrégation des particules.

Les microsphères utilisées dans les poudres non compactées selon l'invention peuvent être réalisées en tous matériaux thermoplastiques non toxiques et non irritants. Ces matériaux peuvent être par exemple des polymères ou copolymères dérivés d'hydrocarbures éthyléniques (par exemple polyéthylène, polystyrène, copolymères chlorure de vinyle-acrylonitrile, etc..) des polyamides, des polyesters, des polymères urée-formaldéhyde, des copolymères d'acrylonitrile et de chlorure de vinylidène, etc...

De préférence, le matériau thermoplastique constituant les microsphères creuses utilisées selon l'invention est un copolymère contenant, en poids, de 20 à 60% de motifs dérivés de chlorure de vinylidène, de 20 à 60% de motifs dérivés d'acrylonitrile, et de 0 à 40% en poids de motifs dérivés d'un monomère acrylique ou styrénique, la somme des pourcentages des ingrédients cités étant égale à 100.

Le monomère acrylique est par exemple un acrylate ou méthacrylate de méthyle ou d'éthyle et de préférence le méthacrylate de méthyle. Le monomère styrénique est par exemple l'alpha-méthylstyrène ou le styrène.

Parmi les microsphères creuses de faible densité obtenues avec ces matériaux, on citera en particulier celles commercialisées sous la marque EXPANCEL par la Société KEMANORD PLAST AB, Sundsvall (Suède).

Les dimensions des microsphères creuses utilisables selon l'invention sont généralement inférieures à 70μm, et de préférence de l'ordre de 20 à 60μm.

Pour réaliser des poudres non compactées selon l'invention, on utilise généralement les microsphères creuses dans des proportions pouvant aller de 0,2 à 20%, et de préférence de 0,5 à 5% en poids par rapport au poids total de la composition.

L'invention a donc pour objet l'utilisation, dans la préparation d'une poudre non compactée pour le maquillage ou les soins du corps et du visage, de microsphères creuses telles que définies ci-dessus, ainsi que les poudres ainsi obtenues.

Les autres ingrédients présents dans les compositions sous forme de poudre non compactée de l'invention sont les ingrédients habituellement utilisés pour la réalisation de ce type de composition.

Parmi ces ingrédients on citera notamment :

- le talc, qui est un silicate de magnésium hydraté, utilisé sous forme de particules généralement de dimensions inférieures à 40μm ; le talc possède des propriétés absorbantes de l'humidité et est utilisé surtout en raison de son toucher onctueux ;
- les micas, qui se présentent sous la forme d'écailles ayant un diamètre de préférence inférieur à 70μm et une épaisseur inférieure à 5μm ; les micas sont des aluminosilicates de compositions variées, qui se présentent sous la forme d'écailles ayant des dimensions de 2 à 200μm, de préférence de 5 à 70μm et une épaisseur de 0,1 à 5μm, de préférence de 0,2 à 3μm. Les micas peuvent être d'origine naturelle (par exemple muscovite, margarite, roscoelithe, lépidolithe, biotite) ou d'origine synthétique. Les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules de forme isotrope ayant des dimensions généralement inférieures à 30μm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 μm dans le cas de l'oxyde de titane) ; ces oxydes ont un toucher onctueux, ont un bon pouvoir couvrant et ont une opacité importante;
- le carbonate de calcium précipité qui, sous forme de particules de dimensions inférieures à 10μm, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium, etc... Ces savons, présents généralement sous la forme de particules ayant des dimensions inférieures à 10μm ont un toucher onctueux et facilitent l'adhérence de la poudre sur la peau ;
- les poudres de polymères synthétiques, tels que le polyéthylène, les polyesters (par exemple isophtalate ou téréphtalate de polyéthylène), les polyamides sous la forme de particules ayant des dimensions inférieures à 50μm, qui possèdent des propriétés absorbantes, et permettent de conférer à la peau un aspect velouté ;
- les huiles, naturelles ou synthétiques, par exemple l'huile de sésame, l'huile de vaseline, la lanoline liquide, les triglycérides de synthèse, l'huile de macadamia, l'huile de jojoba, l'huile d'arara (hoplosthetus), etc... Les huiles accroissent la douceur d'application, favorisent l'adhérence de la poudre sur la peau et permettent de dissoudre certains ingrédients actifs, comme indiqué précédemment. Les poudres de l'invention peuvent contenir jusqu'a 70% en poids d'huile par rapport au poids total de la composition, et le plus souvent de 1,5 à 5% ;
- le stéarate de polyéthylèneglycol et l'alcool cétylique, qui facilitent l'adhérence de la poudre sur la peau ;
- les agents colorants ou nacrants, qui peuvent être constitués par divers pigments colorés, des poudres métalliques (par exemple aluminium ou bronze), etc... ; on peut citer également les micas sur lesquels a été déposée une couche de pigments ou d'agents nacrants tels que par exemple des oxydes de titane, de zirconium, de fer (III), la silice, l'hydroxyde d'aluminium, l'oxychlorure de

EP 0 254 612 B1

bismuth, etc... ; de tels micas modifiés sont décrits par exemple dans les demandes de brevet allemand 3.211.602 et 3.221.045 ;

- des ingrédients actifs divers, tels que des antiseptiques (trichloro diphényl éther, agents cationiques, acide borique, etc...), qui sont utilisés notamment dans les poudres désodorisantes pour le corps ou les pieds et dans les poudres pour bébés ; des agents astringents qui sont utilisés dans les poudres désodorisantes ou dans les poudres pour les pieds tels que l'hydroxychlorure d'aluminium ou les aluns ; des filtres solaires ; des agents adoucissants ; etc...
- des charges comme le carbonate de sodium ;
- des parfums.

En outre, les compositions de poudre de l'invention peuvent être disposées, en association avec un agent propulseur classique, dans un flacon pulvérisateur pour aérosol.

Il faut bien comprendre que tous les ingrédients qui viennent d'être énumérés sont des ingrédients connus largement utilisés dans les divers types de poudres non compactées pour le maquillage ou les soins du corps ou du visage. Le choix de ces ingrédients et la détermination de leurs proportions respectives ne font pas partie de l'invention ; ils sont fonction notamment des approvisionnements, des circonstances économiques, de l'effet recherché, etc... et les critères de ce choix doivent être considérés comme faisant partie des connaissances usuelles des spécialistes.

L'invention concerne notamment une poudre non compactée pour le maquillage ou les soins du corps ou du visage contenant principalement, outre les microsphères de matériau synthétique thermoplastique, des huiles, des poudres de polymères, du mica, du talc, du kaolin ainsi que des savons, des pigments et du parfum.

L'invention concerne en particulier une poudre de maquillage non compactée, ayant la composition pondérale suivante :

- 0,2 à 5% d'un matériau synthétique sous forme de microsphères creuses, telles que définies précédemment,
- 20 à 94% de mica,
- 5 à 45% de poudre de polymères,
- 0 à 4% de savons métalliques,
- 1,5 à 5% d'huile,
- 0 à 30% d'agents nacrants,
- 0 à 5% d'autres ingrédients usuels,
- et des quantités suffisantes de parfum et de pigments, la somme des pourcentages des ingrédients cités étant égale à 100.

Les exemples suivants illustrent l'invention.

EXEMPLE 1

On a préparé une poudre de maquillage non compactée ayant la composition pondérale suivante :

| Mica | 55,45 |
|---|---|
| Orgasol 2002 D | 36 |
| Expancel DE | 1 |
| Myristate de Mg | 2 |
| Huile de vaseline | 3 |
| Pigments : | |
| Oxyde de fer jaune | 1 |
| Oxyde de fer noir | 0,25 |
| DC red 30 | 0,5 |
| Parfum | 0,8 |

4

Les mica en poudre utilisés sont des produits commercialisés par exemple par les sociétés MERCK ou MEARL.

L'Orgasol 2002 D est une marque commerciale pour une poudre de polyamide commercialisée par la société ATO-Chimie.

Expancel DE est la marque commerciale d'une compositon à base de copolymère du type chlorure de vinylidène-acrylonitrile présentée sous la forme de microsphères creuses ayant des dimensions de 40-60$\mu$m, contenant de l'isobutane, ayant une masse spécifique de 0,04g/cm$^3$, commercialisées par la société Kemanord Plast.

Pour préparer cette poudre on a opéré de la façon suivante :

Le mica, l'orgasol, le myristate de Mg et les pigments sont mélangés, puis broyés.

Le broyat est ensuite pulvérisé avec le parfum, puis on ajoute l'huile de vaseline. L'Expancel est finalement additionnée, par simple mélange.

EXEMPLE 2

De façon analogue on a préparé une poudre de maquillage non compactée ayant la composition pondérale suivante :

| | |
|---|---|
| Mica | 46,65 |
| Orgasol 2002 D extra | 28 |
| Expancel DE | 4 |
| Stearate de Zn | 5 |
| Huile d'Arara | 5 |
| Agents nacrants | 10 |
| Pigments : | |
|    Oxyde de fer jaune | 0,4 |
|    Oxyde de fer rouge | 0,3 |
|    Oxyde de fer noir | 0,15 |
| Parfum | 0,5 |

Cette poudre s'applique très facilement et s'étale de façon homogène.
Elle donne un fini parfait, légèrement nacré, au maquillage.

EXEMPLE 3

De façon analogue on a préparé une poudre de maquillage non compactée ayant la composition pondérale suivante :

| Mica | 70,35 |
|---|---|
| Polymist | 20 |
| Expancel DE | 2 |
| Laurate de Zn | 3 |
| Huile de Macadamia | 1,5 |
| Pigments : | |
| Oxyde de fer jaune | 1 |
| Oxyde de fer rouge | 1 |
| Oxyde de fer noir | 0,15 |
| Parfum | 1 |

Polymist est une marque commerciale pour une poudre de polyéthylène commercialisée par la société ALLIED CHEMICAL.

Cette poudre s'applique également très bien et de façon homogène. Elle laisse un fini mat, sans donner de sensation d'inconfort.

EXEMPLE 4 : Exemple comparatif

On a réalisé une poudre non compactée (poudre A) ayant la composition de la poudre de l'exemple 1. Toutefois des pigments différents ont été utilisés pour obtenir 3 teintes : claire, moyenne ou foncée.

On a réalisé 3 autres poudres analogues (poudre B), mais sans microsphères d'Expancel DE.

On a distribué à chacune des 19 utilisatrices ayant participé à ce test une boîte de la poudre avec Expancel DE (de la teinte souhaitée) et une boîte de la poudre de même teinte sans Expancel DE.

Les poudres étaient présentées dans le même conditionnement, et identifiables seulement par les lettre A et B.

Les utilisatrices ont testé librement les poudres A et B pendant 15 jours.

Il a été demandé aux utilisatrices d'indiquer leur préférence, entre les poudres A et B, sur les critères suivants : facilité d'étalement, homogénéité, pouvoir couvrant, effet mat, tenue et confort.

Les résultats ont été les suivants :

10 utilisatrices ont préféré la poudre A ;

7 utilisatrices ont aimé les deux poudres ;

1 utilisatrice n'a pas aimé les poudres ;

1 utilisatrice a préféré la poudre B.

Une préférence statistiquement significative des utilisatrices en faveur de la poudre A a été notée en ce qui concerne la facilité d'étalement, la finesse, la douceur et le confort. Pour les autres critères choisis, aucune préférence significative n'a pu être mise en évidence, ni pour la poudre A, ni pour la poudre B.

EXEMPLE 5 : Poudre libre parfumée pour le corps

On a préparé une poudre ayant la composition pondérale suivante (% en poids) :

EP 0 254 612 B1

| | |
|---|---|
| Stéarate de magnésium | 2 |
| Oxyde de titane | 4 |
| Kaolin | 4 |
| Expancel DE | 2 |
| Huile de jojoba | 2 |
| Talc suprafin | 84 |
| Parfum | 2 |

On introduit dans le mélangeur le stéarate de magnésium, l'oxyde de titane, le kaolin et le talc. Un pré-mélange est effectué par agitation. On pulvérise ensuite progressivement le parfum et l'huile de jojoba sous agitation , puis l'Expancel est additionné, jusqu'à complète homogénéisation.

Cette poudre reste homogène durant son stockage. Elle donne à la peau un toucher doux et confortable.

EXEMPLE 6 : Talc pour bébé sous forme de mousse aérosol

On a préparé la composition suivante (% en poids) :

| | |
|---|---|
| Eau déminéralisée stérile | 38,95 |
| Silice | 0,25 |
| PEG stéarate | 0,5 |
| Alcool cétylique | 0,5 |
| Huile de macadamia | 1,5 |
| Talc | 20 |
| Parfum | 0,3 |
| Ethanol | 35 |
| Expancel DE | 3 |

Remplissage de l'aérosol : 90% de la composition ci-dessus et 10% de butane (en volume).

On mélange le PEG stéarate (stéarate de polyéthylèneglycol), l'alcool cétylique, l'huile de macadamia et on l'ajoute au mélange eau + alcool.

Le talc est ensuite additionné, ainsi que la silice et l'Expancel jusqu'à l'obtention d'une dispersion homogène.

On introduit alors le parfum.

Le mélange est transféré dans un conteneur pour aérosol, qui est serti, puis pressurisé.

Ce talc est d'un emploi facile et il donne un toucher agréable sur la peau du bébé.

EXEMPLE 7 : Poudre anti-transpirante en aérosol

On a préparé la composition suivante (% en poids) :

7

| Talc | 67,7 |
|---|---|
| Expancel DE | 5 |
| Huile de vaseline | 15 |
| Hydroxychlorure d'aluminium | 10 |
| Parfum | 2 |
| Silice colloïdale | 0,3 |

Le propulseur consiste en un mélange 60-40 (vol/vol) de monochlorodifluorométhane et dichlorodifluorométhane.

Remplissage de l'aérosol : 30% de la composition et 70% de propulseur (en volume).

Cet anti-transpirant donne une sensation de fraîcheur lors de l'application, et il freine la transpiration sans tacher les vêtements.

EXEMPLE 8 : Poudre désodorisante pour le corps

On a préparé la composition suivante (% en poids) :

| Talc | 73,3 |
|---|---|
| Stéarate de magnésium | 3 |
| Carbonate de sodium | 2 |
| Expancel DE | 15 |
| Myristate d'isopropyle | 5 |
| Trichlorodiphényléther | 0,2 |
| Parfum | 1,5 |

Cette poudre désodorisante est agréable à l'application, et reste efficace toute la journée.

EXEMPLE 9 : Poudre désodorisante en aérosol pour les pieds

On a préparé la composition suivante (% en poids) :

| Talc | 59,2 |
|---|---|
| Stéarate de magnésium | 5 |
| Expancel DE | 10 |
| Huile de sésame | 20 |
| Hydroxychlorure d'aluminium | 4 |
| Acide borique | 0,5 |
| Parfum | 1 |
| Silice colloïdale | 0,3 |

Le propulseur est identique à celui utilisé dans l'exemple 7.

Remplissage de l'aérosol : 30% de la composition et 70% de propulseur (en volume).

Cette poudre permet d'agir efficacement sur la dégradation de la sueur par les bactéries.

## Revendications

1. Utilisation, dans la préparation d'une poudre non compactée pour le maquillage ou les soins du corps et du visage, de microsphères creuses en matériau synthétique thermoplastique, caractérisée par le fait que lesdites microsphères creuses ont une masse spécifique inférieure à 0,1 g/cm³.

2. Utilisation selon la revendication 1, caractérisée par le fait que ledit matériau thermoplastique est choisi parmi les polymères ou copolymères dérivés d'hydrocarbures éthylénique, les polyamides, les polyesters, les polymères urée-formaldéhyde, et les copolymères d'acrylonitrile et de chlorure de vinylidène.

3. Utilisation selon la revendication 2, caractérisée par le fait que ledit matériau est un copolymère chlorure de vinylidène-acrylonitrile contenant, en poids, de 20 à 60 % de motifs dérivés de chlorure de vinylidène, de 20 à 60 % de motifs dérivés d'acrylonitrile et de 0 à 40 % de motifs dérivés d'un monomère acrylique ou styrénique.

4. Utilisation selon la revendication 3, caractérisée par le fait que ledit monomère acrylique est un acrylate ou méthacrylate de méthyle ou d'éthyle.

5. Utilisation selon la revendication 4, caractérisée par le fait que ledit monomère est le méthacrylate de méthyle.

6. Utilisation selon la revendication 3, caractérisée par le fait que ledit monomère styrénique est le styrène.

7. Utilisation selon l'une quelconque des revendications précédentes, caractérisée par le fait que ladite masse spécifique est inférieure à 0,05 g/cm³.

8. Utilisation selon l'une quelconque des revendications précédentes, caractérisée par le fait que lesdites microsphères creuses ont des dimensions inférieures à 70 $\mu$m.

9. Utilisation selon l'une quelconque des revendications précédentes, caractérisée par le fait que l'on incorpore lesdites microsphères creuses dans des proportions pouvant aller de 0,2 à 20 % et en particulier de 0,5 à 5 % en poids, par rapport au poids total de la composition.

10. Poudre non compactée pour le maquillage ou les soins du corps et du visage, contenant des microsphères creuses de matériau synthétique thermoplastique, caractérisée par le fait que les dites microsphères creuses ont une masse spécifique inférieure à 0,1 g/cm³.

**11.** Poudre selon la revendication 10, caractérisée par le fait que ledit matériau thermoplastique est tel que défini dans l'une quelconque des revendications 2 à 7.

**12.** Poudre selon l'une quelconque des revendications 10 et 11, caractérisée par le fait que lesdites microsphères creuses ont des dimensions inférieures à 70 $\mu$m.

**13.** Poudre selon l'une quelconque des revendications 10 à 12, caractérisée par le fait que lesdites microsphères creuses sont présentes dans des proportions pouvant aller de 0,2 à 20 % et en particulier de 0,5 à 5 % en poids, par rapport au poids total de la composition.

**14.** Poudre selon l'une quelconque des revendications 10 à 13, caractérisée par le fait qu'elle renferme de 1,5 à 10 % en poids d'huile.

**15.** Poudre de maquillage ou pour les soins du corps et du visage selon l'une quelconque des revendications 10 à 14, caractérisée par le fait qu'elle contient principalement, outre les microsphères de matériau synthétique thermoplastique, des huiles, des poudres de polymères, du mica, du talc, du kaolin, ainsi que des savons, des pigments et du parfum.

**16.** Poudre de maquillage selon la revendication 15, caractérisée par le fait qu'elle a la composition pondérale suivante :
- 0,2 à 5 % d'un matériau synthétique sous forme de microsphères creuses,
- 20 à 94 % de mica,
- 5 à 45 % de poudre de polymères,
- 0 à 4 % de savons métalliques,
- 1,5 à 5 % d'huile,
- 0 à 30 % d'agents nacrants,
- 0 à 5 % d'autres ingrédients usuels,
- et des quantités suffisantes de parfum et de pigments, la somme des pourcentages des ingrédients cités étant égale à 100.

## Claims

**1.** Use of hollow microspheres of synthetic thermoplastic material in the preparation of a non-compacted make-up powder for face and body care, characterized by the fact that said hollow microspheres have a specific mass below 0.1g/cm$^3$.

**2.** Use according to claim 1, characterized by the fact that said thermoplastic material is chosen from polymer or copolymers derived from ethylenic hydrocarbons, polyamides, polyesters, urea-formaldehyde polymers and copolymers of acrylonitrile and vinylidene chloride.

**3.** Use according to claim 2, characterized by the fact that said material is a vinylidene chloride-acrylonitrile copolymer containing, by weight, from 26 to 60% of units derived from vinylidene chloride and from 20 to 60% of units derived from acrylonitrile and from 0 to 40% of units derived from an acrylic or styrene monomer.

**4.** Use according to claim 3, characterized by the fact that said acrylic monomer is a methyl or ethyl acrylate or methacrylate.

**5.** Use according to claim 4, characterized by the fact that said monomer is methyl methacrylate.

**6.** Use according to claim 3, characterized by the fact that said styrene monomer is styrene.

**7.** Use according to any one of the preceding claims, characterized by the fact that said specific mass is below 0.05g/cm$^3$.

**8.** Use according to any one of the preceding claims, characterized by the fact that said hollow microspheres are smaller than 70$\mu$m.

EP 0 254 612 B1

9. Use according to any one of the preceding claims, characterized by incorporating from 0.2 to 20%, in particular from 0.5 to 5% by weight of said hollow microspheres related to the total weight of the composition.

10. Non-compacted powder, for make-up or for face and body care, containing hollow microspheres of synthetic thermoplastic material, characterized by the fact that said hollow microspheres have a specific mass below $0.1g/m^3$.

11. Powder according to claim 10, characterized by the fact that said thermoplastic material is as defined in any one of claims 2 to 7.

12. Powder according to any one of claims 10 and 11, characterized by the fact that said hollow microspheres are smaller than $70 \mu m$ .

13. Powder according to any one of claims 10 to 12, characterized by the fact that said hollow microspheres are present in an amount ranging from 0.2 to 20% and in particular from 0.5 to 5% by weight related to the total weight of the composition.

14. Powder according to any one of claims 10 to 13, characterized by the fact that it contains from 1.5 to 70% by weight of oil.

15. Powder for make-up or body acid face care according to any one of claims 10 to 14, characterized by the fact that it contains chiefly, apart from the microspheres of synthetic thermoplastic material, oils, polymer powders, mica, talc, kaolin as well as soaps, pigments and perfume.

16. Powder for make-up according to claim 15 characterized by the fact that it has the following composition by weight :
    - 0.2 to 5% of a synthetic material in the form of hollow microspheres,
    - 20 to 94% of mica,
    - 5 to 45% of polymer powder,
    - 0 to 4% of metallic soaps,
    - 1.5 to 5% of oil,
    - 0 to 30% of nacreous agents,
    - 0 to 5% of other standard ingredients,
    - and sufficient quantities of perfume and pigments, the sum of the percentages of ingredients noted being 100.

## Ansprüche

1. Verwendung von hohlen Mikrokugeln aus synthetischem, thermoplastischem Material zur Herstellung eines nicht-kompaktierten Puders zum Schminken oder zur Körper- und Gesichtspflege, dadurch gekennzeichnet, daß das spezifische Gewicht der Mikrokugeln kleiner als $0,1g/cm^3$ ist.

2. Verwendung nach Anspruch 1, dadurch gekennzeichnet, daß das thermoplastische Material ausgewählt ist unter Polymeren und Copolymeren, welche von Ethylenkohlenwasserstoff abgeleitet sind, Polyamiden, Polyestern, Harnstoff-Formaldehyd-Polymeren und Copolymeren von Acrylnitril und Vinylidenchlorid.

3. Verwendung nach Anspruch 2, **dadurch gekennzeichnet,** daß das Material ein Vinylidenchlorid - Acrylnitril-Copolymer ist, das 20 bis 60 Gew.- % von Vinylidenchlorid abgeleitete Einheiten, 20 bis 60 Gew.-% von Acrylnitril abgeleitete Einheiten und 0 bis 40 Gew.-% von einem Acryl- oder Styrolmonomer abgeleitete Einheiten enthält.

4. Verwendung nach Anspruch 3, **dadurch gekennzeichnet,** daß das Acrylmonomer ein Methyl- oder Ethylacrylat oder -methacrylat ist.

5. Verwendung nach Anspruch 4, **dadurch gekennzeichnet,** daß das Monomer Methylmethacrylat ist.

11

6. Verwendung nach Anspruch 3, **dadurch gekennzeichnet,** daß das Styrolmonomer Styrol ist.

7. Verwendung nach einem der vorhergehenden Ansprüche, **dadurch gekennzeichnet,** daß das spezifische Gewicht kleiner als 0,05 g/cm$^3$ ist.

8. Verwendung nach einem der vorhergehenden Ansprüche, **dadurch gekennzeichnet,** daß die hohlen Mikrokugeln kleiner als 70 μm sind.

9. Verwendung nach einem der vorhergehenden Ansprüche, **dadurch gekennzeichnet,** daß die hohlen Mikrokugeln in einem Anteil von 0,2 bis 20 Gew.-% und insbesondere von 0,5 bis 5 Gew.-%, bezogen auf das Gesamtgewicht des Mittels, zugegeben werden.

10. Nicht-kompaktierter Puder zum Schminken oder zur Körper-und Gesichtspflege, enthaltend hohle Mikrokugeln aus synthetischem, thermoplastischem Material, **dadurch gekennzeichnet,** daß die hohlen Mikrokugeln ein spezifisches Gewicht von unter 0,1 g/cm$^3$ aufweisen.

11. Puder nach Anspruch 10, **dadurch gekennzeichnet,** daß das thermoplastische Material ausgewählt ist unter Material gemäß der Definition einer der Ansprüche 2 bis 7.

12. Puder nach einem der Ansprüche 10 und 11, **dadurch gekennzeichnet,** daß die hohlen Mikrokugeln kleiner als 70 μm sind.

13. Puder nach einem der Ansprüche 10 bis 12, **dadurch gekennzeichnet,** daß die hohlen Mikrokugeln in einem Anteil von 0,2 bis 20 Gew.−% und insbesondere von 0,5 bis 5 Gew.−%, bezogen auf das Gesamtgewicht des Mittels, enthalten sind.

14. Puder nach einem der Ansprüche 10 bis 13, **dadurch gekennzeichnet,** daß er 1,5 bis 70 Gew.-% Öl enthält.

15. Puder zum Schminken oder zur Körper- und Gesichtspflege nach einem der Ansprüche 10 bis 14, **dadurch gekennzeichnet,** daß er außer den Mikrokugeln aus synthetischem, thermoplastischem Material im wesentlichen Öle, pulverförmige Polymere, Mica, Talk, Kaolin sowie Seifen, Pigmente und Parfum enthält.

16. Puder zum Schminken nach Anspruch 15, **dadurch gekennzeichnet** daß er folgende auf das Gewicht bezogene Zusammensetzung besitzt:

```
        -   0,2 - 5 % eines synthetischen Materials in
                    Form hohler Mikrokugeln,
        -  20 -  94 % Mica,
        -   5 -  45 % pulverförmige Polymere,
        -   0 -   4 % Metallseifen,
        -   1,5 - 5 % Öl,
        -   0 -  30 % Perlmuttglanz verleihende Mittel
        -   0 -   5 % andere, übliche Ingredienzien
```

- und ausreichende Mengen an Parfum und Pigmenten, wobei die prozentuale Summe der genannten Ingredienzien gleich 100 ist.